# FASCICULE DE BREVET EUROPEEN

(11) **EP 0 995 442 B1**
(45) Date de publication et mention de la délivrance du brevet: **28.01.2009**
(21) Numéro de dépôt: 99117855.9
(22) Date de dépôt: 10.09.1999
(51) Int. Cl.: A61K 35/20, A61K 9/00, A61P 1/02

(54) **Utilisation du lait d'équidés pour la fabrication d'une préparation destinée au traitement des troubles bucco-dentaires**
Verwendung von Pferdemilch zur Herstellung einer Zusammensetzung zur Behandlung von Mund- und Zahnstörungen
Use of milk from equines for the manufacture of a preparation for the treatment of bucco-dental troubles

(30) Priorité: 15.09.1998 FR 9811598
(43) Date de publication de la demande: 26.04.2000
(73) Titulaire: LE PETIT Leon, Antoine, Georges, William, 57720 Hottviller (FR)
(72) Inventeur: Le Petit, Léon, Antoine, Georges, William, F-57720 Hottviller (FR); Rollan, Sergi, F-09100 Pamiers (FR)
(74) Mandataire: Vièl, Christof

(56) Documents cités:
- EP-A- 0 140 498
- EP-A- 0 743 060
- WO-A-94/16675
- WO-A-95/01100
- WO-A-98/00149
- WO-A-98/51316
- AT-B- 393 961
- Butler-JE International Dairy Federation; Indigenous Antimicrobial Agents of Milk - Recent Developments 2. Passive Immunity and immunoglobulin diverstiy; p 14-50
- "2. Therapeutiques et prevention; ISBN 285598-7091 mp 124-127" EDITION INSERM * pages 124-125 *
- CLARK-WB ET AL.: "Mechanisms of initiation and progression of periodontal disease" PERIODONTOLOGY 2000, vol. 2, - 1993 pages 72-82, ISBN 0906-6713
- GOODSON-JM: "Patterns of progression and regression of advanced destructive periodontal disease" JOURNAL OF CLINICAL PERIODONTOLOGY, vol. 9, 1982, page 472-481,
- Dr. S Rollan 20/02/2001 (not published); 4 pages Etude exploratoire de la stimulation salivaire par les cachets de lait de jument Jum'Vital
- LOPEZ JORNET P ET AL.: "Desordnes del fluido salivial: hiposecretion e hipersecretion salival." MEDICINA ORAL; PREVALENCIA Y DISTRIBUCION DE LAS CANDIDOSIS ORALES EN PACI, vol. 1, 1996, page 96-106, ISBN 1137-2834
- BANDERAS-TARABAY-JA ET AL.: "Flujo y concentracion de proteinas en saliva total humana" SALUD PUBLICA DE MEXICO, vol. 39, no. 5, octobre 1997 (1997-10), page 433-441,
- ORTEGA PANTALEON-ME ET AL.: "Evaluacion del flujo y viscosidad salival y su relacion con el indice de caries" MEDISAN, vol. 2, no. 2, 1998, page 33-39,
- Faculte d'Odontologie, Universite de Rennes 1; Questions d'internat en Parodontologie; Question n° 100, Indices paradonteaux et indices de carie. Utilisation. Importance. Perrin-M et al. (7 pages)
- L'instiut babock pour la recherche et le development international du secteur latier; Universite du Wisconsin a Madison; Essentiels Laitiers Elevage des genisses - de la naissance au sevrage 2) importance de nourrir le nuveau-ne avec du colostrum Wattiaux-MA (5 pages)
- HLULMANN-H: "1 page" 1991, ATLAS DE POCHE DE PHARMACOLOGIE
- ROITT-I: "Immunologie fondamentale et appliquee" MEDSI MCGRAW-HILL
- http://www.alimentation-france.com/home/De couvrir/index.asp?Id=8&Rubrique=1 Le lait de transition décrit que vers le 3ème jour, le colostrum fait place au lait de transition...Lorsque le 5ème jour arrive, le lait de transition devient encore plus clair et c'est là le lait définitif..
- http://www.moormans.com/dairy/DairyFF/dair ymar98/colostru.htm
- Pschyrembl Klinisches Wörterbuch 258. Auflage; Walter de Gruyter, 1998; page 1061 - 1062 "Muttermilch".

## Description

La présente invention concerne l'utilisation du lait de jument pour la fabrication d'une préparation destinée à une nouvelle application thérapeutique.

L'utilisation du lait de jument et du lait d'ânesse dans l'alimentation et en médecine a une tradition déjà très ancienne.

Les grandes caractéristiques du lait de jument et d'ânesse étaient bien connues en France au siècle dernier. Ce lait était réservé à tous ceux qui souffraient de troubles digestifs, biliaires ou pancréatiques (Zhangabylov A.K, Salkhanov B.A 1977, 3 Voprosy Pitaniya). Il était également conseillé aux adultes atteints de tuberculose pulmonaire et de certaines maladies pulmonaires chroniques (bronchites, asthme ...), dans les maladies gastro-intestinales (ulcères, entérites ...), dans les maladies hépatiques chroniques (hépatites, cirrhoses ...), dans les maladies cardiaques (insuffisance, artériosclérose, hypertension ...) et dans les maladies métaboliques (diabète ...) (Lozovich en 1995 dans Culture Dairy Product 30, 18-21 *sous le titre « Medical uses of whole and fermented mare milk in Russia »).*

Le lait et le colostrum d'équidés et de ruminant ont fait l'objet ces dernières années de nombreuses demandes de brevet.

C'est ainsi que le document WO 98 01100 présente l'utilisation du lait d'ânesse en application topique sur la peau, notamment comme tenseur de la peau en cosmétique.

Le document WO 94 16675 présente l'utilisation de colostrum d'équidés mélangé à du lait d'équidés pour des traitements externe de la peau, notamment en cas de brûlures, de lésions de contact et de maladies cutanées. La présence de lait n'est considérée que comme une option.

Dans le document WO 98 00149, le colostrum d'équidés additionné de lait en poudre est utilisé pour le traitement des maladies inflammatoires intestinales et les dysfonctionnements anorectaux.

Dans le document EP 0 140 498, les mammifères femelles, notamment des juments, sont immunisés lors de la gestation avec un antigène spécifique et le colostrum est prélevé directement après la naissance du petit. Le colostrum ainsi récolté contient un anticorps qui est extrait du lait pour, associé à un composé synergiste, entrer dans la composition d'une préparation destinée à la prévention des caries dentaires. L'anticorps agit directement sur les bactéries à l'origine de la formation de la plaque dentaire responsable des caries. Il est envisagé de ne pas extraire l'anticorps mais d'utiliser directement le colostrum récolté. Dans cet exemple, les propriétés pharmacologiques intrinsèques du colostrum ne sont pas utilisées. Cette méthode est relativement complexe et peu respectueuse de l'animal.

Le colostrum de ruminants a été également utilisé dans le domaine de l'hygiène buccale, comme le montre le document EP 0 743 060.

Il est important ici de souligner la différence qui existe entre le colostrum et le lait. Le colostrum est un liquide visqueux sécrété par les glandes mammaires durant les premiers jours qui suivent la naissance. Il précède la formation du lait dont il se distingue par sa composition.

L'inconvénient de l'utilisation du colostrum réside notamment dans le fait qu'il est prélevé au détriment du petit animal qui vient juste de naître et qui a besoin de cet aliment aux propriétés pharmacologiques particulières. De plus, le colostrum n'est produit que quelques jours, ce qui le rend d'autant plus précieux.

Les propriétés naturelles du lait ne se conservent pas longtemps et sont très sensibles à la chaleur. C'est pourquoi, des méthodes de conservation ont été mises au point. Dans le document WO 98 01100 cité précédemment, le lait d'ânesse est congelé entre -18 et -40 °C puis il est décongelé directement avant application. Dans le document AT 393 961, le lait de jument est transformé en lait en poudre par évaporation après ajout de dioxyde de silicium. Le lait en poudre peut ainsi être utilisé pour des préparations orales, qui ne sont d'ailleurs pas spécifiées.

Le colostrum est un produit riche en anticorps mais qui ne peut être récolté qu'en faible quantité pendant un laps de temps très court, et ce au détriment du petit qui vient de naître.

Par ailleurs, le colostrum, qu'il soit immunisé (EP 0 140 498) ou non (EP 0 743 060), et l'extrait de colostrum (EP 0 743 060) agissent par l'intermédiaire du transfert d'anticorps (sérothérapie) et stimulent l'immunité à médiation humorale spécifique. Cette immunité passive n'a qu'un effet curatif provisoire : lorsqu'il n'y a plus d'anticorps, il n'y a plus d'effet (Butler, JE (1994), passive immunity and immunoglobulin diversity. In: Indigenous Antimicrobial Agents of Milk - Récent Developments. International Dairy Fédération Special Issue 9404, 14-50).

De plus les auteurs de EP 0 743 060 précisent que le colostrum serait beaucoup plus efficace que le lait normal en raison sa teneur en IgG particulièrement importante (page 3, lignes 29-31).

Par ailleurs, tous les troubles bucco-dentaires ne sont pas d'origine immunitaire et la seule stimulation du système immunitaire ne peut régler tous ces maux (Goadson et coll 1982, Pattern of progression and regression of advanced destructive periodontal disease, J. Clin. Periodontal 9 : 472-481).

L'objectif de l'invention est donc de fournir une préparation qui ait un effet curatif aussi bien que préventif, de manière prolongée.
Cet objectif est atteint par l'utilisation du lait d'équidés pour la fabrication d'une préparation pour application dans la cavité buccale destinée au traitement et à la prévention des troubles bucco-dentaires. Le lait d'équidés est utilisé notamment pour des préparations destinées à stimuler la salivation. En effet, le lait d'équidés aide la régulation de la sécrétion salivaire et améliore la qualité de la salive. Le lait d'équidés s'est avéré particulièrement efficace dans le traitement et la prévention des aphtes, des caries dentaires, des gingivites, des parodontoses et des stomatites. Il peut donc être utilisé dans les préparations destinées au traitement et à la prévention du syndrome de Gougerot-Sjögren.

Il s'agit ici d'utiliser exclusivement du lait d'équidés sans ajout de colostrum. C'est d'ailleurs en cela que la présente demande se différencie de la demande de brevet WO 98 51316 publiée après la date de priorité de cette demande, et qui utilise du colostrum d'équidés additionné de lait.

Les utilisations du lait d'équidés précédemment citées concernent des domaines thérapeutiques très éloignés du domaine bucco-dentaire. Le lait d'équidés agit sur des cellules différentes selon des processus qui ne peuvent pas être comparés.

Par ailleurs, le colostrum et le lait, qu'ils soient d'équidés ou de ruminants, se différencient par leurs compositions et leurs propriétés chimico-physiques et pharmacologiques.

Le pH du colostrum, inférieur à 6,3, est proche de celui de la salive. Il n'agit donc pas sur le système tampon de la salive et ne stimule donc pas la salivation. Il est donc en particulier inefficace dans le traitement du syndrome de Gougerot-Sjögren.

Le colostrum est plus riche que le lait en matières azotées, en particulier en albumines, dont 80 % sont des d'immunoglobulines, et en matières minérales, surtout en chlorures, mais il est moins riche en lactose.

De plus, le lait d'équidés ne contient qu'environ 10 % de matières sèches tandis que le lait de ruminants (vaches, chèvres, brebis) en contient 30 % et le colostrum plus de 60 %.

Ces différences de composition rendent le lait d'équidés beaucoup plus hydrosoluble que le lait de ruminants ou que le colostrum. Ceci se traduit par une meilleure absorption du lait d'équidés au niveau des muqueuses bucco-linguales et perlinguales et par une meilleure imprégnation de la salive. La salive étant bien imprégnée des éléments essentiels du lait d'équidés, l'action de ceux-ci est prolongée.

Par ailleurs, le mode d'action du colostrum est différent de celui du lait.

Le colostrum est plus acide que le lait. Le pH du colostrum, même celui d'équidés, est inférieur à 6,3, il agit comme auto-nettoyeur de la voie entérale (bouche, intestin, rectum). Le colostrum ne modifie pas la salivation. Le lait d'équidés par contre à un pH neutre compris entre 7,1 et 7,3 et il agit sur le système tampon de la salive. Il provoque notamment sa stimulation et augmente le rôle du lait dans le traitement et la prévention des affections bucco-dentaires.

De plus, le colostrum, en raison en particulier de son acidité et de la présence d'enzymes et d'immunoglobulines, a un effet antiseptique direct contre les troubles bucco-dentaires ce qui tend à diminuer la plaque dentaire et à stimuler l'immunité. Il agit également sur des récepteurs spécifiques. Cet effet est dû à des substances non présentes normalement dans l'organisme. Il n'agit cependant pas sur la salivation.

Le lait, en particulier celui d'équidés, a au contraire un effet antiseptique indirect contre les troubles bucco-dentaires. Cet effet, dû à des substances intervenant normalement dans le fonctionnement de l'organisme, provoque une stimulation des fonctions salivaires.

Par conséquent, l'utilisation de lait d'équidés à la place de colostrum de ruminants entraîne une meilleure absorption des principes essentiels du lait par la salive et les muqueuses de la bouche. De plus, le prélèvement de lait, en général lorsque le petit est en voie de sevrage, ne se fait pas à son détriment.

Pour avoir une action efficace, il est nécessaire que le lait d'équidés reste suffisamment longtemps dans la cavité buccale. Il existe alors plusieurs voies d'application possibles. Le gargarisme ne permet guère de laisser agir le lait d'équidés sur l'ensemble de la cavité buccale ; il vise principalement le fond de la bouche et l'entrée de la gorge. Il est par contre possible de conserver quelques minutes une gorgée de lait d'équidés dans la bouche, le laissant ainsi agir sur l'ensemble de la cavité buccale et imprégner la salive. Cependant, cette application est relativement contraignante, car elle interdit pratiquement tout mouvement et surtout toute conversation.

Conformément à l'invention, il est possible de contourner cet inconvénient en utilisant le lait d'équidés non pas tel quel mais sous forme de lyophilisat. La lyophilisation est une technique qui permet de conserver l'intégrité du produit lorsque les conditions de lyophilisation optimales sont respectées. Placé sous la langue, le lyophilisat de lait de jument se dissout dans la salive qui ensuite est répartie uniformément dans l'ensemble de la cavité buccale.

Pour faciliter l'application du lyophilisat de lait de jument, l'invention propose de le présenter sous une forme galénique le libérant lentement. Ce peut être par exemple un cachet azyme qui, mis sous la langue de préférence le soir avant le coucher, se désagrège pour libérer le lyophilisat. Une autre solution consiste à présenter le lyophilisat sous forme de comprimé. Dans ce cas, il faudra faire attention lors de la fabrication des comprimés à ne pas provoquer un échauffement trop important du lyophilisat afin de ne pas risquer une perte partielle des éléments essentiels qui le constituent. Le cachet devra être sucé lentement, de préférence le soir en se couchant. Une troisième solution réside dans la présentation sous forme de pâte à mâcher. Ici encore, les conditions de fabrication, et notamment l'échauffement du lyophilisat, devront être contrôlées de façon sévère. Le lyophilisat peut également être présenté sous forme de sirop qu'on laissera agir dans la bouche.

Le lait d'équidés ainsi dissous dans la salive contribue à revitaliser le pouvoir tampon de celle-ci et à empêcher la fuite du calcium dentaire. La teneur élevée en lysozyme du lait d'équidés associée à la vitamine C également contenue dans ce lait explique vraisemblablement le rôle de défense de la cavité buccale, pour assurer la protection de la muqueuse et combattre les microbes.

Quelques exemples d'application non limitatifs sont décrits ci-dessous.

### Cachet azyme :

Lyophiliser le lait d'équidés selon un procédé connu. Enfermer 400 mg de lyophilisat ainsi obtenu dans un cachet azyme en pâte d'amidon de maïs (par ex. fourni par LGA à Bandol). Le cachet est mis dans la bouche, de préférence au coucher, où il se désagrège en libérant lentement le lyophilisat de lait d'équidés.

### Sirop :

Dissoudre 200 g de lyophilisat de lait d'équidés dans 680 g d'eau tiède, puis ajouter 1 120 g de sucre qui seront dissous à feux doux. Filtrer le sirop ainsi préparé et le mettre en bouteille hermétique. La posologie conseillée est d'une cuillérée à dessert de sirop 4 fois par jour, à laisser agir dans la bouche.

### Pâte à mâcher:

Dissoudre 1 000 g de gomme arabique dans un litre d'eau chaude, puis filtrer la solution afin de retenir les impuretés. Ajouter 1 000 g de sirop de lait d'équidés obtenu selon le procédé décrit ci-dessus, mélanger à feux doux. Couler ces pâtes de gomme arabique sous la forme voulue. On mâchera six à dix pâtes dans la journée.

L'utilisation du lait d'équidés pour la fabrication d'une préparation destinée à la prévention et au traitement des troubles bucco-dentaires telle qu'elle est décrite précédemment est particulièrement respectueuse de l'environnement et de l'animal. Elle est de plus sans danger pour le patient.

## Revendications

1. Utilisation du lait d'équidés, pour la fabrication d'une préparation pour application dans la cavité buccale destinée au traitement et à la prévention des troubles bucco-dentaires, le pH du lait d'équidé étant compris entre 7,1 et 7,3.

2. Utilisation du lait d'équidés selon la revendication 1, **caractérisée en ce que** la préparation est destinée à stimuler la salivation.

3. Utilisation du lait d'équidés selon la revendication 2, **caractérisée en ce que** la préparation est destinée au traitement et à la prévention des affections bucco-dentaires.

4. Utilisation du lait d'équidés selon la revendication 3, **caractérisée en ce que** la préparation est destinée au traitement et à la prévention des aphtes, de la gingivite, de la parodontose, de la stomatite ou des caries dentaires.

5. Utilisation du lait d'équidés selon l'une des revendications précédentes, **caractérisée en ce que** le lait d'équidés est présent sous forme de lyophilisat.

6. Utilisation du lait d'équidés selon la revendication 5, **caractérisée en ce que** le lyophilisat entre dans la composition d'une forme galénique libérant lentement le lyophilisat dans la cavité buccale.

7. Utilisation du lait d'équidés selon la revendication 6, **caractérisée en ce que** le lyophilisat est incorporé dans un cachet azyme.

8. Utilisation du lait d'équidés selon la revendication 6, **caractérisée en ce que** le lyophilisat est incorporé dans un comprimé.

9. Utilisation du lait d'équidés selon la revendication 6, **caractérisée en ce que** le lyophilisat est incorporé dans une pâte à mâcher.

10. Utilisation du lait d'équidés selon la revendication 6, **caractérisée en ce que** le lyophilisat est incorporé dans un sirop.

## Claims

1. Use of equine milk for the manufacture of a preparation for application in the buccal cavity intended for the treatment and prevention of mouth disorders, the equine milk pH being between 7.1 and 7.3.

2. Use of equine milk according to claim 1, **characterised in that** the preparation is intended to stimulate salivation.

3. Use of equine milk according to claim 2, **characterised in that** the preparation is intended for the treatment and prevention of mouth or teeth disorders.

4. Use of equine milk according to claim 3, **characterised in that** the preparation is intended for the treatment and prevention of mouth ulcers, gingivitis, periodontal disease, stomatitis and dental caries.

5. Use of equine milk according to one of the preceding claims, **characterised in that** the equine milk is present in the form of a freeze-dried substance.

6. Use of equine milk according to claim 5, **characterised in that** the freeze-dried substance forms part of the composition of a galenic form slowly releasing the freeze-dried substance in the buccal cavity.

7. Use of equine milk according to claim 6, **characterised in that** the freeze-dried substance is incorporated in an azyme cachet.

8. Use of equine milk according to claim 6, **characterised in that** the freeze-dried substance is incorporated in a tablet.

9. Use of equine milk according to claim 6, **characterised in that** the freeze-dried substance is incorporated in a paste for chewing.

10. Use of equine milk according to claim 6, **characterised in that** the freeze-dried substance is incorporated in a syrup.

## Patentansprüche

1. Verwendung der Milch von Einhufern für die Herstellung einer Zubereitung zur Anwendung in der Mundhöhle, welche für die Behandlung und Vorbeugung von Mund- und Zahnproblemen bestimmt ist, wobei der pH-Wert der Milch von Einhufern zwischen 7,1 und 7,3 liegt.

2. Verwendung der Milch von Einhufern gemäß Anspruch 1, **dadurch gekennzeichnet, dass** die Zubereitung zur Stimulierung des Speichelflusses dient.

3. Verwendung der Milch von Einhufern gemäß Anspruch 2, **dadurch gekennzeichnet, dass** die Zubereitung für die Behandlung und Vorbeugung von Mund- und Zahnerkrankungen bestimmt ist.

4. Verwendung der Milch von Einhufern gemäß Anspruch 3, **dadurch gekennzeichnet, dass** die Zubereitung für die Behandlung und Vorbeugung von Aphten, Gingivitis, Parodontose, Stomatitis und Zahnkaries bestimmt ist.

5. Verwendung der Milch von Einhufern gemäß einem der vorausgegangenen Ansprüche, **dadurch gekennzeichnet, dass** die Milch von Einhufern in Form eines Lyophilisats vorliegt.

6. Verwendung der Milch von Einhufern gemäß Anspruch 5, **dadurch gekennzeichnet, dass** das Lyophilisat in die Zusammensetzung einer galenischen Form eingeht, welche das Lyophilisat langsam in der Mundhöhle freisetzt.

7. Verwendung der Milch von Einhufern gemäß Anspruch 6, **dadurch gekennzeichnet, dass** das Lyophilisat in einer Oblate enthalten ist.

8. Verwendung der Milch von Einhufern gemäß Anspruch 6, **dadurch gekennzeichnet, dass** das Lyophilisat in einer Tablette enthalten ist.

9. Verwendung der Milch von Einhufern gemäß Anspruch 6, **dadurch gekennzeichnet, dass** das Lyophilisat in einer Kaupaste enthalten ist.

10. Verwendung der Milch von Einhufern gemäß Anspruch 6, **dadurch gekennzeichnet, dass** das Lyophilisat in einem Sirup enthalten ist.
